# EUROPEAN PATENT SPECIFICATION

(11) **EP 4 368 597 B1**
(45) Date of publication and mention of the grant of the patent: **05.11.2025**
(21) Application number: 23200343.4
(22) Date of filing: 28.09.2023
(51) Int. Cl.: C04B 35/488

(54) **ZIRCONIA-BASED ANTIBACTERIAL DENTURE MATERIAL AND PREPARATION METHOD THEREOF**
ANTIBAKTERIELLES ZAHNPROTHESENMATERIAL AUF ZIRKONOXIDBASIS UND HERSTELLUNGSVERFAHREN DAFÜR
MATÉRIAU DE PROTHÈSE DENTAIRE ANTIBACTÉRIEN À BASE DE ZIRCONE ET SON PROCÉDÉ DE PRÉPARATION

(30) Priority: 11.11.2022 CN 202211412647
(43) Date of publication of application: 15.05.2024
(73) Proprietor: Shenzen Yurucheng Dental Materials Co., Ltd., Shenzhen, Pingshan 518000 (CN)
(72) Inventor: LI, Zongyu, Shenzhen, 518000 (CN); LIU, Wei, Shenzhen, 518000 (CN); ZHANG, Yu, Shenzhen, 518000 (CN); LIU, Jianjun, Shenzhen, 518000 (CN)
(74) Representative: Monteiro Alves, Inês

(56) References cited:
- JP-A- H0 833 650
- US-A1- 2013 014 671
- US-B2- 10 723 657

## Description

### TECHNICAL FIELD

The present application relates to the technical field of denture, in particular to a zirconia-based antibacterial denture material and a preparation method thereof.

### BACKGROUND

Dentures, commonly referred to as "false teeth", are a general term in medicine for the prosthetic restorations made to replace partially or completely missing teeth in the upper and lower jaws. Dentures are categorized into two types: removable and fixed. Fixed dentures cannot be removed and worn by patients themselves, while removable dentures can be easily taken in and out by the patients.

For breaking the balance of the original oral ecological environment, the dentures as foreign bodies in the oral cavity, are more likely to hide dirt than natural teeth. If they are not cleaned in time, plaque will form on the surfaces of the dentures and natural teeth. If acid bacteria remain on the enamel surface for a long time, it can cause oral diseases such as dental caries, gingivitis, periodontal disease, and denture stomatitis. In more severe cases, other pathogenic bacteria attached to the dentures may enter a respiratory system through an oropharynx. There is also a risk of causing infectious diseases such as bacterial pneumonia. Therefore, there is an urgent need for antibacterial denture materials with antibacterial properties to solve the problem of existing dentures that break the oral ecological balance and cause oral diseases after wearing them.

In the existing technology, two methods are usually used for antibacterial treatment: denture surface treatment and self-treatment. The former is to change the charge on the surface of the denture or form a polyelectrolyte multilayer film, etc. However, during the use of this method, due to long-term wear of the denture, its antibacterial properties can be reduced. The latter is to add antibacterial agents such as zinc oxide during the denture molding process. However, since a band gap of zinc oxide is 3.3ev, it can only absorb ultraviolet light with a wavelength less than 385nm, and cannot exert its antibacterial effect very well when used in visible light or dark environments. Thus, the antibacterial effect of this type of antibacterial denture also needs to be improved.

US 10723657 discloses a sintered product having a chemical analysis such that, as percentages by weight on the basis of the oxides, ZrO₂ partially stabilized with CeO₂ and Y₂O₃: balance to 100%, Al₂O₃: > 10% and < 19% additive chosen from CaO, manganese oxides, ZnO, praseodymium oxides, SrO, copper oxides, Nd₂O₃, BaO, iron oxides, and mixtures thereof: 0.2-6%, impurities: <2%, CeO₂ and Y₂O₃ being present in amounts such that, as a molar percentage on the basis of the sum of ZrO₂, CeO₂ and Y₂O₃CeO₂: ≥2.5 mol. % and <5.5 mol. % Y₂O₃: 0.5-2 mol. %.

### SUMMARY

The present application provides a zirconia-based antibacterial denture material and a preparation method thereof. The zirconia-based antibacterial denture material has a good antibacterial effect and high strength.

In order to achieve the above technical objective, the present application adopts the following technical solutions.

The present application provides a zirconia-based antibacterial denture material, including components with the following mass parts: 75 to 80 parts of zirconium oxide, 2 to 5 parts of nanosized silver oxide, 3 to 5 parts of nanosized zinc oxide, 1 to 3 parts of nanosized lanthanum oxide, 1 to 3 parts of nanosized yttrium oxide, 1 to 2 parts of cerium oxide, and 1 to 2 parts of size control agent.

The size control agent is calcium silicate.

In some embodiments, a ratio of a sum of masses of the silver oxide and the zinc oxide to the mass of the yttrium oxide is between 5 to 0.5 and 5 to 1.

In some embodiments, a particle size of the zirconium oxide is less than or equal to 30 nm.

The present application further provides a preparation method of a zirconia-based antibacterial denture material, including:
S1, weighing zirconium oxide, silver oxide, zinc oxide, lanthanum oxide, yttrium oxide, cerium oxide and size control agent in proportion, and mixing by ball milling, to obtain ceramic powder;
S2, putting the ceramic powder into a mold and pre-pressing the ceramic power to obtain an antibacterial denture porcelain block precursor;
S3, performing cold isostatic pressing, pre-sintering, and high-temperature sintering on the antibacterial denture porcelain block precursor to obtain the zirconia-based antibacterial denture material.

In some embodiments, a pressure of the cold isostatic pressing is 100 MPa to 300 MPa, and a pressure holding time is 60 seconds to 600 seconds.

In some embodiments, a pressure of the pre-pressing is 5 MPa to 50 MPa, and a pressure holding time is 10 seconds to 60 seconds.

In some embodiments, a final temperature of the pre-sintering is 800°C to 1000°C, a heating rate is 10°C/min to 15°C/min and a temperature holding time is 1 hour to 5 hours.

In some embodiments, a final temperature of the high-temperature sintering is 1400°C to 1550°C, a heating rate is 1°C/min to 10°C/min and a temperature holding time is 1 hour to 5 hours.

In some embodiments, a process of the high-temperature sintering is performed in a magnetic field environment.

The beneficial effects of the present application are as follows: in this technical solution, by mixing and sinthering zinc oxide, lanthanum oxide, and cerium oxide, the zirconia-based antibacterial denture material has excellent antibacterial properties, and can effectively inhibit escherichia coli and staphylococcus aureus for a long time. The addition of lanthanum oxide, yttrium oxide, cerium oxide and size control agents in the zirconia-based antibacterial denture material overcomes the defect of reducing the mechanical properties of the zirconium oxide ceramic caused by adding antibacterial materials, such as zinc oxide and silver oxide. The preparation method of a zirconia-based antibacterial denture material is simple and fast, and by adjusting the pressure and sintering parameters, it can prevent "porcelain chipping" during the molding process.

### BRIEF DESCRIPTION OF THE DRAWINGS

In order to more clearly illustrate the technical solutions in the embodiments of the present application or in the related art, drawings in the embodiments or in the related art will be briefly described below. Obviously, the drawings in the following description are only some embodiments of the present application. Other drawings can be obtained by those skilled in the art according to the structures shown in the drawings without creative efforts.

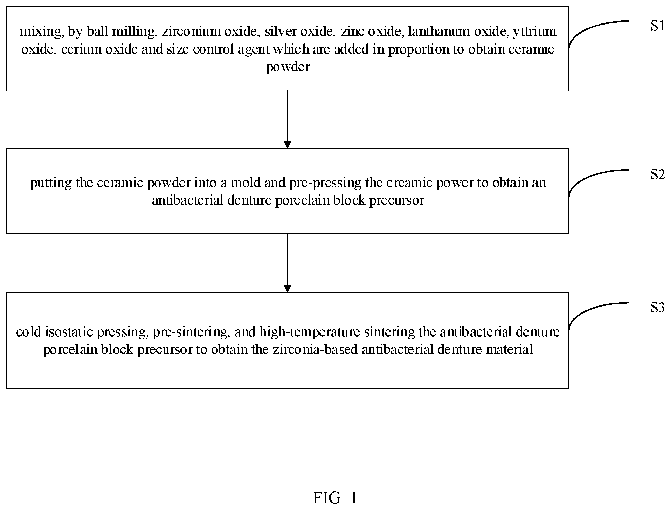

FIG. 1 is a schematic flowchart of a preparation method for a zirconia-based antibacterial denture material according to some embodiments of the present application.

The realization of the purpose, functional characteristics and advantages of the present application will be further described with reference to the attached drawings in combination with embodiments.

### DETAILED DESCRIPTION OF THE EMBODIMENTS

In order to clarify the purpose, technical solutions and advantages of the present application, the present application will be further described in detail below in conjunction with embodiments. It should be understood that the specific embodiments described here are only used to explain the present application and are not intended to limit the present application.

As a conventional antibacterial material, zinc oxide has been generally recognized in antibacterial ceramics, but it is rarely used in denture ceramics. The reason is that a band gap of zinc oxide is 3.3ev and can only absorb ultraviolet light with a wavelength less than 385nm. However, the use environment of dentures is mostly hot, humid and dark or visible light conditions. Therefore, zinc oxide cannot fully exert its antibacterial properties in denture materials. Although silver oxide is also a conventional antibacterial material, its utility as a denture material is not applicable, since its stability and durability are poor and the denture material needs to be used for a long time. Furthermore, zirconia-based denture materials need to have excellent mechanical properties to prevent denture damage, and adding soft metal-based oxides such as silver oxide, zinc oxide, copper oxide and other substances can reduce their wear resistance. Therefore, it is necessary to provide a zirconia-based antibacterial denture material that can not only ensure the wear resistance of zirconia-based dentures but also improve their antibacterial performance.

Based on this, the present application is proposed.

The present application provides a zirconia-based antibacterial denture material, including components with the following mass parts: 75 to 80 parts of zirconium oxide, 2 to 5 parts of nanosized silver oxide, 3 to 5 parts of nanosized zinc oxide, 1 to 3 parts of nanosized lanthanum oxide, 1 to 3 parts of nanosized yttrium oxide, 1 to 2 parts of cerium oxide, 1 to 2 parts of size control agent.

In this technical solution, in order to improve the absorption of visible light by zinc oxide, other modified ions can be doped to change and narrow the band gap of zinc oxide, while increasing surface defects to improve the photocatalytic activity of zinc oxide, and thereby enhancing the antibacterial ability. After sintering, cerium and lanthanum in the zirconia-based antibacterial denture material synergistically promote the formation of zinc oxide in the nanosized wurtzite crystal form to reduce a bandgap width value, thereby improving its photocatalytic antibacterial activity. At the same time, lanthanum oxide itself also has antibacterial properties and can cooperate with other antibacterial substances to enhance the antibacterial rate and broad spectrum. Cerium oxide can also form a reinforced composite material with zirconium oxide to improve the strength of the material. Meanwhile, lanthanum oxide has a strong restriction effect on the grains growth of zinc oxide. Under the restriction effect, more silver can enter the zinc oxide crystal lattice to act as an electron acceptor and change the semiconductor energy level, causing zinc oxide to produce more antibacterial active centers, and zinc oxide with a special structure in the product has been proven to produce reactive oxygen species such as hydroxyl radicals, superoxide anions, hydrogen peroxide and other reactive oxygen species to kill bacteria. Zinc oxide do not change or decrease during this process. At the same time, nano-effects in the local or hybrid structures of special structure zinc oxide antibacterial agent with the special structure, as well as the synergistic effect of trace amounts of zinc ions within a controlled range, can help to enhance their bactericidal performance. The bactericidal mechanism is enhanced through the synergy of active oxygen effects, nano-effects, ionic effects, etc., and ultimately improve the antibacterial ability of zirconia-based denture materials.

In addition, lanthanum oxide can increase the sintering density of zirconia-based ceramics, but its sintering temperature is relatively high. Therefore, adding yttrium oxide reduces its sintering temperature and improves its sintering performance. At the same time, lanthanum oxide and yttrium oxide can synergistically enhance the strength of zirconia-based ceramics, and improve its wear resistance. Although yttrium oxide also has this function, the concentration of single-component yttrium oxide is too high, which is bad for mechanical properties. That's why lanthanum oxide and yttrium oxide are added together to improve the strength of denture materials. Therefore, it's necessary for yttrium oxide and lanthanum oxide to work together to increase the density and strength of the material to form a dense and smooth denture.

Among the above components, the size control agent is calcium silicate to control the excessive expansion of the material during the molding process and to prevent the occurrence of pores or porcelain chipping.

A ratio of a sum of the masses of silver oxide and zinc oxide to the mass of yttrium oxide is between 5:0.5 and 5: 1. Excessive yttrium oxide can reduce the mechanical strength of the denture material, but within this range, it can be ensured that the strength of the denture material can not decrease due to the addition of sliver oxide or zinc oxide.

The particle size of zirconium oxide is less than or equal to 30 nm. As the base material, the zirconium oxide used is nanosized zirconium oxide. This particle size is beneficial to the mixing uniformity of the material during the preparation process.

The present application further provides a preparation method of a zirconia-based antibacterial denture material, as shown in FIG. 1, including the following steps:
S1, weighing zirconium oxide, silver oxide, zinc oxide, lanthanum oxide, yttrium oxide, cerium oxide and size control agent in proportion, and mixing by ball milling to obtain ceramic powder;
S2, putting the ceramic powder into a mold and pre-pressing the ceramic power to obtain an antibacterial denture porcelain block precursor; and
S3, performing cold isostatic pressing, pre-sintering, and high-temperature sintering treatment on the antibacterial denture porcelain block precursor in sequence to obtain the zirconia-based antibacterial denture material.

A pressure of the cold isostatic pressing is 100MPa to 300MPa. In some embodiments, the pressure of the cold isostatic pressing is 200 MPa to 300 MPa, with a pressure holding time of 60 seconds to 600 seconds; and a pre-pressing pressure is 5 MPa to 50 MPa. In some embodiments, the pre-pressing pressure is 40 MPa to 50 MPa, with a pressure holding time of 10 seconds to 60 seconds.

The final temperature of the pre-sintering is 800°C to 1000°C, with a heating rate of 10°C/min to 15°C/min. In some embodiments, the heating rate is 10°C/min to 15°C/min and a temperature holding time is 1 hour to 5 hours. The final temperature of the high-temperature sintering is 1400°C to 1550°C, with a heating rate of 1°C/min to 10°C/min. In some embodiments, the heating rate is 1°C/min to 3°C/min and the temperature holding time is 1 hour to 5 hours. Under this program, the sintering efficiency can be guaranteed and collapsing or cracking of the porcelain caused by rapid heating can be prevented.

In some embodiments, the high-temperature sintering process is carried out in a magnetic field environment, which can improve the uniform distribution of each material component, not only enhancing the effective antibacterial performance in all directions of the material without leaving any hygienic dead corners, but also improving the mechanical strength of the material.

The present application will be further described below through specific embodiments.

### Embodiments 1 to 7

A zirconia-based antibacterial denture material, including the following components: zirconium oxide, nanosized silver oxide, nanosized zinc oxide, nanosized lanthanum oxide, nanosized yttrium oxide, cerium oxide and calcium silicate. The formulation of components of the zirconia-based antibacterial denture material of embodiments 1-7 is shown in Table 1.

**Table 1: the formulation of components of the zirconia-based antibacterial denture material of embodiments**

| | Zirconium oxide | Nanosized silver oxide | Nanosized zinc oxide | Nanosized lanthanum oxide | Nanosized yttrium oxide | Cerium oxide | Calcium silicate |
|---|---|---|---|---|---|---|---|
| Embodiment 1 | 75 | 5 | 5 | 3 | 2 | 1 | 2 |
| Embodiment 2 | 75 | 5 | 5 | 3 | 2 | 1.5 | 2 |
| Embodiment 3 | 75 | 5 | 5 | 3 | 2 | 2 | 2 |
| Embodiment 4 | 75 | 5 | 5 | 2 | 2 | 2 | 2 |
| Embodiment 5 | 75 | 5 | 5 | 1 | 2 | 2 | 2 |
| Embodiment 6 | 80 | 5 | 5 | 1 | 3 | 2 | 1 |
| Embodiment 7 | 80 | 2 | 3 | 3 | 2 | 2 | 1 |

The zirconia-based antibacterial denture material is prepared according to the components in Table 1, the preparation steps are as follows:
S1, weighing zirconium oxide, silver oxide, zinc oxide, lanthanum oxide, yttrium oxide, cerium oxide and size control agent in proportion, and mixing by ball milling, to obtain ceramic powder;
S2, putting the ceramic powder into a mold and pre-pressing the ceramic power to obtain an antibacterial denture porcelain block precursor, wherein the pre-pressing pressure is 5 MPa to 50 MPa and the pressure holding time is 10 seconds to 60 seconds; and
S3, performing cold isostatic pressing on the antibacterial denture porcelain block precursor, the pressure of the cold isostatic pressing is 100 MPa to 300 MPa and the pressure holding time is 60 seconds to 600 seconds; then performing the pre-sintering on the antibacterial denture porcelain block precursor, the temperature of the pre-sintering is 800°C to 1000°C, a heating rate is 10°C/min and a temperature holding time is 1 hour to 5 hours; finally, performing the high-temperature sintering in the magnetic field strength of 8T, the temperature of the high-temperature sintering is 1400°C to 1550°C, a heating rate is 1°C/min and a temperature holding time is 1 hour to 5 hours, to obtain the zirconia-based antibacterial denture material.

### Embodiment 8

A zirconia-based antibacterial denture material, including the following components: 75 parts of zirconium oxide, 5 parts of nanosized silver oxide, 5 parts of nanosized zinc oxide, 3 parts of nanosized lanthanum oxide, 2 parts of nanosized yttrium oxide, 2 parts of cerium oxide and 2 parts of calcium silicate.

The preparation steps of the zirconia-based antibacterial denture material are as follows:
S1, weighing zirconium oxide, silver oxide, zinc oxide, lanthanum oxide, yttrium oxide, cerium oxide and size control agent in proportion, and mixing by ball milling, to obtain ceramic powder;
S2, putting the ceramic powder into a mold and pre-pressing the ceramic power to obtain an antibacterial denture porcelain block precursor, wherein the pre-pressing pressure is 5 MPa to 50 MPa and the pressure holding time is 10 seconds to 60 seconds; and
S3, performing the cold isostatic pressing the antibacterial denture porcelain block precursor, the pressure of the cold isostatic pressing is 100 MPa to 300 MPa and the pressure holding time is 60 seconds to 600 seconds; then performing the pre-sintering on the antibacterial denture porcelain block precursor, the final temperature of the pre-sintering is 800°C to 1000°C, with the heating rate of 10°C/min and the temperature holding time of 1 hour to 5 hours; finally, performing the high-temperature sintering on the antibacterial denture porcelain block precursor, the temperature of the high-temperature sintering is 1400°C to 1550°C, with the heating rate of 1°C/min and the temperature holding time of 1 hour to 5 hours, to obtain the zirconia-based antibacterial denture material.

### Comparative Examples 1 to 6

The raw material components and preparation processes in Comparative Examples 1 to 6 are substantially the same as those in Embodiment 3. The difference is that in Comparative Examples 1-6, the components: nanosized silver oxide, nanosized zinc oxide, nanosized lanthanum oxide, and nanosized yttrium oxide, cerium oxide and calcium silicate are not added in sequence.

### Testing and Evaluation

The antibacterial properties and bending strength of the zirconia-based antibacterial denture materials in Embodiments 1 to 8 and Comparative Examples 1 to 6 were tested.

Antibacterial performance measurement: putting the test sample and blank control sample into sterilized petri dishes respectively, taking 100 µL of bacterial suspension by a micro-sampler and dropping the bacterial suspension on the surface of each sample, and making three parallel samples for each group. Using a sterile tweezers to pick up the polyethylene film and cover it on the surface of each sample; laying it flat without any bubbles to make the bacterial solution evenly contact the surface of the sample; covering the plate and contact culturing it for 24 hours in a environment of (37±1)°C and a relative humidity of 90% under natural light or humid and dark conditions; for the samples after contact culture, using 20 mL of physiological saline eluent, repeatedly wash the sample and covering film three times (using tweezers to pick up the film and rinsing); mixing the eluate thoroughly evenly, diluting 10 times to obtain appropriate colony forming units (CFU) count, inoculating 2 plate culture media in parallel for each 100 µL sample solution, and counting the colony after incubation under the conditions of (37±1)°C and relative humidity of 90%. Finally, calculating the actual number of recovered colonies of the sample according to the dilution factor. The above experiment was repeated three times and the average value was taken. The formula for calculating the antibacterial rate is: R(%)=(A-B)/A× 100%, wherein: R represents antibacterial rate (%), A represents the average number of colonies recovered from the blank control sample (CFU/mL), and B represents the average number of recovered colonies (CFU/mL) in the test example. The antibacterial rate results are shown in Table 2.

The flexural strength of each zirconia-based antibacterial denture material was tested according to the method in GBT 6569-2006. The results are shown in Table 2.

**Table 2: Test results of antibacterial properties and flexural strength**

| | Escherichia coli antibacterial rate (%) | | Staphylococcus aureus antibacterial rate (%) | | Flexural strength(/Mpa) |
|---|---|---|---|---|---|
| | natural light condition | dark and moist condition | natural light condition | dark and moist condition | |
| Embodiment 1 | 88 | 81 | 86 | 82 | 183 |
| Embodiment 2 | 92 | 85 | 93 | 86 | 185 |
| Embodiment 3 | 99 | 88 | 99 | 90 | 187 |
| Embodiment 4 | 97 | 82 | 96 | 84 | 193 |
| Embodiment 5 | 93 | 79 | 94 | 80 | 201 |
| Embodiment 6 | 96 | 84 | 95 | 86 | 174 |
| Embodiment 7 | 98 | 87 | 98 | 90 | 160 |
| Embodiment 8 | 98 | 87 | 98 | 90 | 183 |
| Comparative Example 1 | 65 | 60 | 62 | 59 | 177 |
| Comparative Example 2 | 70 | 64 | 71 | 68 | 181 |
| Comparative Example 3 | 78 | 80 | 85 | 73 | 161 |
| Comparative Example 4 | 98 | 86 | 98 | 89 | 143 |
| Comparative Example 5 | 72 | 75 | 82 | 81 | 153 |
| Comparative Example 6 | 99 | 86 | 97 | 88 | 151 |

Compared with Embodiment 3, the high-temperature sintering process of Embodiment 6 is not carried out under magnetic field conditions. As can be seen from Table 2, the magnetic field is conducive to the improvement of antibacterial performance and flexural strength. Compared with Embodiment 3, the temperature rising of sintering in Embodiment 7 is accelerated, which reduces the mechanical properties of the material. In Embodiments 1 to 3, the content of cerium oxide is adjusted, and in Embodiments 3 to 5, the content of nanosized lanthanum oxide is adjusted. It can be seen that, under natural light condition, cerium oxide has a greater influence on the antibacterial effect than lanthanum oxide. Increasing the proportion of cerium oxide is more conducive to improving the antibacterial rate. However, under dark condition, lanthanum oxide has a greater influence on the antibacterial effect than cerium oxide. Increasing the proportion of lanthanum oxide is more conducive to improving the antibacterial rate, and the increase of lanthanum oxide is conducive to the flexural strength of the material. Compared with Embodiment 3, the high-temperature sintering is not carried out under the magnetic field in Embodiment 8, it can be seen that its mechanical properties are reduced. Taking Embodiment 3 as a reference, in Comparative Examples 1 to 6, nanosized silver oxide, nanosized zinc oxide, nanosized lanthanum oxide, nanosized yttrium oxide, cerium oxide, and calcium silicate are respectively not added, it can be seen that nanosized silver oxide, nanosized zinc oxide, nanosized lanthanum oxide and cerium oxide have a synergistic antibacterial effect, while lanthanum oxide, cerium oxide, yttrium oxide and calcium silicate are beneficial to improve the flexural strength of zirconia-based ceramic materials.

## Claims

1. A zirconia-based antibacterial denture material, **characterized by** comprising components with the following mass parts: 75 to 80 parts of zirconium oxide, 2 to 5 parts of nanosized silver oxide, 3 to 5 parts of nanosized zinc oxide, 1 to 3 parts of nanosized lanthanum oxide, 1 to 3 parts of nanosized yttrium oxide, 1 to 2 parts of cerium oxide, and 1 to 2 parts of size control agent, the size control agent is calcium silicate to control the excessive expansion of the material during the molding process and to prevent the occurence of pores or porcelain chipping.

2. The zirconia-based antibacterial denture material according to claim 1, wherein a ratio of a sum of masses of the silver oxide and the zinc oxide to the mass of the yttrium oxide is between 5 to 0.5 and 5 to 1.

3. A preparation method of a zirconia-based antibacterial denture material according to any one of claims 1 to 2, **characterized by** comprising:
(S1), weighing zirconium oxide, silver oxide, zinc oxide, lanthanum oxide, yttrium oxide, cerium oxide and size control agent in proportion, and mixing by ball milling, to obtain ceramic powder;
(S2), putting the ceramic powder into a mold and pre-pressing the ceramic power to obtain an antibacterial denture porcelain block precursor;
(S3), performing cold isostatic pressing, pre-sintering, and high-temperature sintering on the antibacterial denture porcelain block precursor to obtain the zirconia-based antibacterial denture material.

4. The preparation method of the zirconia-based antibacterial denture material according to claim 3 , wherein a pressure of the cold isostatic pressing is 100 MPa to 300 MPa, and a pressure holding time is 60 seconds to 600 seconds.

5. The preparation method of the zirconia-based antibacterial denture material according to claim 3
, wherein a pressure of the pre-pressing is 5 MPa to 50 MPa, and a pressure holding time is 10 seconds to 60 seconds.

6. The preparation method of the zirconia-based antibacterial denture material according to claim 3
, wherein a final temperature of the pre-sintering is 800°C to 1000°C, a heating rate is 10°C/min to 15°C/min and a temperature holding time is 1 hour to 5 hours.

7. The preparation method of the zirconia-based antibacterial denture material according to claim 3
, wherein a final temperature of the high-temperature sintering is 1400°C to 1550°C, a heating rate is 1°C/min to 10°C/min and a temperature holding time is 1 hour to 5 hours.

8. The preparation method of the zirconia-based antibacterial denture material according to claim 3
, wherein a process of the high-temperature sintering is performed in a magnetic field environment.

## Patentansprüche

1. Antibakterielles Zahnersatzmaterial auf Zirkonoxidbasis, **dadurch gekennzeichnet, dass** es Komponenten mit den folgenden Masseanteilen umfasst: 75 bis 80 Teile Zirkoniumoxid, 2 bis 5 Teile nanoskaliges Silberoxid, 3 bis 5 Teile nanoskaliges Zinkoxid, 1 bis 3 Teile nanoskaliges Lanthanoxid, 1 bis 3 Teile nanoskaliges Yttriumoxid, 1 bis 2 Teile Ceroxid und 1 bis 2 Teile Größenkontrollmittel, wobei das Größenkontrollmittel Calciumsilikat ist, um die übermäßige Ausdehnung des Materials während des Formgebungsprozesses zu kontrollieren und das Auftreten von Poren oder Porzellanabplatzungen zu verhindern.

2. Antibakterielles Zahnersatzmaterial auf Zirkoniumoxidbasis nach Anspruch 1, wobei das Verhältnis der Summe der Massen von Silberoxid und Zinkoxid zur Masse von Yttriumoxid zwischen 5 zu 0,5 und 5 zu 1 liegt.

3. Herstellungsverfahren eines antibakteriellen Zahnersatzmaterials auf Zirkoniabasis gemäß einem der Ansprüche 1 bis 2, **dadurch gekennzeichnet, dass** es umfasst:
(S1), Abwiegen von Zirconiumoxid, Silberoxid, Zinkoxid, Lanthanoxid, Yttriumoxid, Ceroxid und Größenkontrollmittel im Verhältnis und Mischen durch Kugelmühlen, um Keramikpulver zu erhalten;
(S2), Einbringen des Keramikpulvers in eine Form und Vorpressen des Keramikpulvers, um einen antibakteriellen Zahnprothesen-Porzellanblock-Vorläufer zu erhalten;
(S3), Durchführen von kaltem isostatischen Pressen, Vorsintern und Hochtemperatursintern des antibakteriellen Zahnprothesen-Porzellanblock-Vorläufers, um das antibakterielle Zahnersatzmaterials auf Zirkoniabasis zu erhalten.

4. Herstellungsverfahren eines antibakteriellen Zahnersatzmaterials auf Zirkoniabasis nach Anspruch 3, wobei der Druck des kalten isostatischen Pressens 100 MPa bis 300 MPa beträgt und die Druckhaltezeit 60 Sekunden bis 600 Sekunden beträgt.

5. Herstellungsverfahren eines antibakteriellen Zahnersatzmaterials auf Zirkoniabasis nach Anspruch 3, wobei der Druck des Vorpressens 5 MPa bis 50 MPa beträgt und die Druckhaltezeit 10 Sekunden bis 60 Sekunden beträgt.

6. Herstellungsverfahren eines antibakteriellen Zahnersatzmaterials auf Zirkoniabasis nach Anspruch 3, wobei die Endtemperatur des Vorsinterns 800°C bis 1000°C beträgt, die Heizrate 10°C/min bis 15°C/min beträgt und die Temperaturhaltezeit 1 Stunde bis 5 Stunden beträgt.

7. Herstellungsverfahren eines antibakteriellen Zahnersatzmaterials auf Zirkoniabasis nach Anspruch 3, wobei die Endtemperatur des Hochtemperatursinters 1400°C bis 1550°C beträgt, die Heizrate 1°C/min bis 10°C/min beträgt und die Temperaturhaltezeit 1 Stunde bis 5 Stunden beträgt.

8. Herstellungsverfahren eines antibakteriellen Zahnersatzmaterials auf Zirkoniabasis nach Anspruch 3, wobei ein Prozess des Hochtemperatursinterns in einer Magnetfeldumgebung durchgeführt wird.

## Revendications

1. Matériau de prothèse dentaire antibactérienne à base de zircone, **caractérisé en ce qu'**il comprend des composants de parts en masse suivantes : 75 à 80 parts d'oxyde de zirconium, 2 à 5 parts d'oxyde d'argent de taille des particules nanométrique, 3 à 5 parts d'oxyde de zinc de taille des particules nanométrique, 1 à 3 parts d'oxyde de lanthane de taille des particules nanométrique, 1 à 3 parts d'oxyde d'yttrium de taille des particules nanométrique, 1 à 2 parts d'oxyde de cérium et 1 à 2 parts d'agent de contrôle de taille des particules, l'agent de contrôle de taille des particules est le silicate de calcium pour contrôler l'expansion excessive du matériau pendant le processus de moulage et pour éviter l'apparition de pores ou d'écaillage de porcelaine,

2. Matériau de prothèse dentaire antibactérienne à base de zircone selon la revendication 1, dans lequel un rapport d'une masse totale de l'oxyde d'argent et de l'oxyde de zinc à la masse de l'oxyde d'yttrium est compris entre 5 à 0,5 et 5 à 1.

3. Procédé de préparation d'un matériau de prothèse dentaire antibactérienne à base de zircone selon l'une quelconque des revendications 1 à 2, **caractérisé en ce qu'**il comprend les étapes suivantes :
(S1), peser en proportion l'oxyde de zirconium, l'oxyde d'argent, l'oxyde de zinc, l'oxyde de lanthane, l'oxyde d'yttrium, l'oxyde de cérium et l'agent de contrôle de taille des particules, et mélanger par broyage à billes pour obtenir une poudre céramique ;
(S2), mettre la poudre céramique dans un moule et pré-presser la poudre céramique pour obtenir un précurseur de bloc de porcelaine de prothèse dentaire antibactérienne ;
(S3), effectuer un pressage isostatique à froid, un pré-frittage et un frittage à haute température sur le précurseur de bloc de porcelaine de prothèse dentaire antibactérienne pour obtenir le matériau de prothèse dentaire antibactérienne à base de zircone,

4. Procédé de préparation du matériau de prothèse dentaire antibactérienne à base de zircone selon la revendication 3, dans lequel une pression du pressage isostatique à froid est de 100 MPa à 300 MPa, et une durée de maintien de la pression est de 60 secondes à 600 secondes.

5. Procédé de préparation du matériau de prothèse dentaire antibactérienne à base de zircone selon la revendication 3, dans lequel une pression du pré-pressage est de 5 MPa à 50 MPa, et une durée de maintien de la pression est de 10 secondes à 60 secondes.

6. Procédé de préparation du matériau de prothèse dentaire antibactérienne à base de zircone selon la revendication 3, dans lequel une température finale du pré-frittage est de 800°C à 1000°C, une vitesse de chauffage est de 10°C/min à 15°C/min et une durée de maintien de la température est de 1 heure à 5 heures.

7. Procédé de préparation du matériau de prothèse dentaire antibactérienne à base de zircone selon la revendication 3, dans lequel une température finale du frittage à haute température est de 1400°C à 1550°C, une vitesse de chauffage est de 1°C/min à 10°C/min et une durée de maintien de la température est de 1 heure à 5 heures.

8. Procédé de préparation du matériau de prothèse dentaire antibactérienne à base de zircone selon la revendication 3, dans lequel un processus du frittage à haute température est effectué dans un environnement de champ magnétique.
